(19) Europäisches Patentamt
European Patent Office
Office européen des brevets

(11) Publication number: **0 483 919 A2**

# EUROPEAN PATENT APPLICATION

(21) Application number: **91202771.1**

(22) Date of filing: **24.10.91**

(51) Int. Cl.5: **C07C 29/151**, C07C 31/04

(30) Priority: **29.10.90 GB 9023465**
**29.10.90 GB 9023466**

(43) Date of publication of application:
**06.05.92 Bulletin 92/19**

(84) Designated Contracting States:
**BE DE FR GB IT NL**

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **Sie, Swan Tiong**
**Badhuisweg 3**
**NL-1031 CM Amsterdam(NL)**

(54) Process for the production of methanol.

(57) Methanol is prepared in a process characterised in that a gaseous mixture comprising hydrogen and carbon monoxide is reacted in the presence of a catalyst in a plurality of sequentially arranged stages, the gaseous mixture contacting the catalyst in each stage in a fluidized bed regime whilst being cooled, in which process methanol is removed from the reaction mixture between successive stages. The interstage removal of methanol is preferably carried out by cooling the effluent stream from the preceding reactor and allowing condensation of the methanol to occur. Cooling of the effluent stream is preferably effected by heat exchange with cold feed gas, more preferably using a heat exchanger having a specific heat transfer area of at least 150 $m^2/m^3$, preferably of at least 200 $m^2/m^3$.

EP 0 483 919 A2

The present invention relates to a process for the production of methanol, in which a gaseous mixture comprising hydrogen and carbon monoxide is reacted in the presence of a catalyst composition in a fluidized bed.

Processes for the production of methanol by reacting a gaseous mixture comprising hydrogen and carbon monoxide in the presence of a catalyst composition are known in the art. For example, processes employing the catalyst in the form of a fixed bed, a slurry, or in a fluidized bed regime have been disclosed in the prior art.

Ind. Eng. Chem. Res. 1989, 28, 763-771 discloses a process for the synthesis of methanol in a high pressure miniplant comprising two packed tubular reactor stages, with a high-temperature interstage removal of methanol being effected by absorption at reaction temperatures in a packed bed with tetraethylene glycol dimethylether as the solvent. This process, however, suffers the disadvantage that the effluent gas from the absorption stage may become saturated with solvent vapour at the temperature and pressure prevailing in the synthesis stage, resulting in capillary condensation of the liquid in the catalyst particles.

A lecture reporting work by Y. Saito, M. Kuwa and O. Hashimoto during the 1987 Annual Meeting of the American Institute of Chemical Engineers, New York, November 15-20, 1978, entitled "Development of a fluidized-bed methanol synthesis process" discloses a process using a reactor with the catalyst in a fluidized bed, cooled by means of a cooling jacket covering the surrounding wall of the bed. The temperature of the reaction was adjusted by the varying temperature of the coolant system, in which system water was converted into high pressure steam. The process required high gas space velocities, resulting in a considerable drop in pressure over the reactor. The conversion per pass achieved was about 16%. Unconverted carbon monoxide and hydrogen were recompressed and recycled through the reactor.

A lecture reporting work by M.F.M. Post; S.T. Sie and J.M. Oelderik during the Chemeca'88 (Australia's Bicentennial International Conference for the process industries), Sydney 28-31 August, 1988, entitled "Synthesis of methanol in a fluidized bed of catalyst" disclosed a fluidized bed methanol synthesis process operated at bench scale achieving conversions of up to 60% at a reaction pressure of 8.1 MPa and a reaction temperature of 250 °C (523 K). Good catalyst stability was observed. However, the conversions achieved were too low for operation of the process on a commercial scale.

Further, Chemical Week, 36 (April 16, 1980) discloses a process referred to as "Chem Systems' three-phase process" in which an inert liquid is used to fluidize the catalyst and to remove the heat of reaction. Good conversion figures per pass are allegedly achievable in this process. However, the reliance of the process on an inert liquid led to transport problems and affected the reaction rate. The process further required the separation of methanol from the entrained inert liquid.

Cheap methanol in very large quantities is a valuable product, useful as a fuel and a starting material for further chemical processing. Accordingly, there is a need for an economically attractive, industrially applicable process, utilising cheap starting materials.

The formation of methanol from hydrogen and carbon monoxide is a highly exothermic equilibrium reaction. Thus, at the relatively high operating pressures and temperatures required for reasonable reaction rates, the attainable conversion is strongly limited by the thermodynamic equilibrium. Finding a satisfactory compromise between reaction rate and conversion is, therefore, difficult. Effective control of the reaction temperature across the catalyst bed has proved to be especially important.

In industrially applied processes, in which the catalyst is present in the form of a fixed bed of particles, high gas velocities are applied to promote effective removal of heat and to allow the reaction temperature to be controlled. As a result of these high velocities and the thermodynamic limitations discussed above, only low conversions per pass are obtained. Typical conversion figures are less than 30 %. To achieve acceptable yields of methanol from a mixture of carbon monoxide and hydrogen, it is customary to recompress unconverted feed gas and recycle it to the reactor inlet. This requires recycle compressors of large capacities, which are costly and have a high power consumption.

According to the present invention, there is provided a process for the preparation of methanol, characterised in that a gaseous mixture comprising hydrogen and carbon monoxide is reacted in the presence of a catalyst in a plurality of sequentially arranged stages, the gaseous mixture contacting the catalyst in each stage in a fluidized bed regime whilst being cooled, in which process methanol is removed from the reaction mixture between successive stages.

The process of the present invention allows methanol to be produced on a commercial scale in high yields, whilst utilising relatively simple process configurations and equipment. In particular, the process may advantageously be operated without the need for the compression and recycle of unconverted feed gas, whilst still achieving the required high yields of methanol.

The gaseous feed mixture may comprise hydrogen and carbon monoxide in any suitable molar ratio. The feed mixture preferably comprises hydrogen and carbon monoxide in a molar ratio $H_2/CO$ in the range of from 1 to 3, more preferably from 1.5 to 2.5. A most suitable hydrogen to carbon monoxide molar ratio for the feed is about 2:1.

The gaseous feed mixture may be obtained by any of the processes well known in the art, for example the partial oxidation of methane with oxygen (yielding a mixture having a hydrogen to carbon monoxide ratio of about 2:1), or by the reforming of methane and/or carbon dioxide (yielding a mixture having a hydrogen to carbon monoxide ratio of about 3:1). Gaseous mixtures produced by the partial oxidation of methane are particularly preferred.

The operating temperatures of the synthesis stages will depend on the activity of the catalyst composition employed. The operating temperature may range from about 100 to 350 °C, more preferably from about 200 to about 320 °C.

The operating pressures of the methanol synthesis stages will again be dependent on the activity of the catalyst composition employed and typically range from about 5 to 35 MPa, more preferably from 5 to 15 MPa. With particularly active catalysts, pressures below 10 MPa may be used.

Most advantageously, the operating conditions of the process may be selected so as to achieve conversions of at least 50% per stage, more preferably, the conditions are selected to yield conversions of above 75% per stage.

The fluidized catalyst bed of each stage is cooled to remove heat released during the synthesis reaction. Means for cooling fluidized catalyst beds are known in the art and comprise, for example, coiled tubes arranged within the reactor vessel. Preferably, the reactor has a specific heat transfer area below 1,500 $m^2/m^3$.

The catalyst compositions employed in the process of this invention may be any of the methanol synthesis catalysts known in the art. Examples of suitable catalysts include compositions comprising copper and zinc, optionally promoted with another element such as aluminium or chromium, and compositions comprising a noble metal, for example palladium and/or platinum. The catalyst composition may also comprise a suitable carrier, such as carbon, silica and/or alumina. Catalyst compositions comprising copper and zinc, optionally promoted with another element are preferred.

The particle size and particle size distribution of the catalyst material must be suitable for operation in the fluidized bed regime. Suitable catalyst particles may be obtained by grinding and sieving oversize particles of catalyst. A particle size below 0.4 mm is generally preferred for catalysts for use in the process of the present invention.

The interstage removal of methanol between the successive stages of the process of the present invention may be achieved, for example, by the cooling and condensation of methanol from the effluent of the synthesis stage, or by adsorption/absorption of the methanol from the effluent to a suitable solvent. Processes in which the methanol is removed between successive stages by means of cooling and condensation are preferred. The cooling/condensation and removal of the methanol may be effected by passing the effluent of the synthesis stage through a heat exchanger and, thereafter, to a gas/liquid separator. The gaseous phase from which methanol has been condensed and removed may then be used directly as the feed for the subsequent synthesis stage, without the need for reheating or recompression.

In a particularly preferred arrangement, methanol is removed by cooling the effluent of the reactor in a heat exchanger by indirect heat exchange with cold feed gas for the process. This arrangement is preferably employed to effect each interstage removal of methanol. The heat exchange between the effluent of a reactor and the feed gas may be effected by at using least one heat exchanger having a specific heat transfer area of at least 150, preferably at least 200 $m^2/m^3$. Such heat exchangers, which combine a large exchange surface area with a compact construction, are already known in the art, albeit for different purposes. Particular designs of heat exchanger which are suitable for use in the process include spiral-wound (or coiled) heat exchangers and plate-fin heat exchangers, described in more detail in "Liquefied Natural Gas by W.L.Lom, Applied Science Publishers Ltd, London 1974", pages 56 and 57. In order to be suitable for the practice of the present invention, the heat exchanger should be enclosed in a shell capable of resisting a pressure difference of between 5 and 35 MPa. Thin-walled tubing or plates used in combination with relatively low pressure shells offer the advantage that much less construction material is required for the same heat exchange duty, as compared with conventional heat exchanger designs.

In a preferred embodiment, a spiral wound heat exchanger is employed, in which the reactor effluent is cooled in countercurrent with the incoming feed gas, which acts as a coolant. More preferably, the spiral wound heat exchanger comprises several bundles of spiral wound tubes, each bundle comprising a separate inlet and outlet and being used to cool or heat different streams, thus combining several heat exchange duties in a single vessel.

A preferred scheme for the process of the present invention is one employing a series of synthesis stages in combination with the interstage removal of methanol, in which each subsequent synthesis stage has a lower capacity than that of the previous synthesis stage. Such a process scheme may be effected by using a single reactor in each stage, with the capacity of the reactor decreasing with each successive stage of the process. Alternatively, each stage may comprise a group of reactors arranged in parallel, with the number of reactors, and hence the capacity, of each stage decreasing with successive stages of the process. The operation of the process in this preferred scheme provides the advantage that the need for the recycle of unconverted feed gas is obviated, thereby allowing the process to tolerate higher concentrations of inert components, for example methane, in the feed. This has the advantage that, if partial oxidation is used as the source for the hydrogen/carbon monoxide feed mixture, the purity of the oxygen required in the oxygen feed to the partial oxidation unit may be relaxed.

A particularly preferred reactor configuration for use in either one or more of the synthesis stages of the process comprises a single reactor vessel housing a plurality of interconnected fluidized bed sections arranged in series, in which each fluidized section is cooled by at least one heat exchanger. The configuration is preferably operated such that the temperature of the final fluidized bed section encountered by the feed gas is maintained below the highest temperature of the preceding sections. Preferably, the cooling duty of each heat exchanger in the configuration is arranged to provide a temperature profile through the reactor in which the feed mixture comprising hydrogen and carbon monoxide encounters each successive fluidized bed section at a lower temperature than the preceding section. The cooling medium used to cool the first section(s) of the configuration encountered by the feed gas is preferably water, most preferably present as a boiling liquid to thus raise steam. Subsequent sections of the configuration are preferably cooled using cold feed gas as the cooling medium,

The reactor configuration is typically operated so as to have the maximum temperature of the fluidized bed sections in the range of from 200 to 350 °C, preferably from 250 to 320 °C. The temperature in the final fluidized bed section is typically in the range of from 10 to 100 °C, preferably at least 25 °C, lower than the highest temperature achieved in the preceding sections.

Steam generated in the first section(s) of the fluidized catalyst bed may be used for purposes outside the present process. The cooling medium of the subsequent section(s) is preferably the incoming feed gas, which is preheated, preferably by means of a feed gas pre-heater located in the reactor, prior to use in the process itself.

It will be appreciated that, a typical arrangement of the preferred reactor configuration comprises a vertical reactor vessel, with the feed gas entering the lower portion of the vessel. In such an arrangement, the first sections of the fluidized bed encountered by the feed gas will be the lower sections. The later, or final, sections of the bed encountered by the gas will be the highest sections, or those situated in the upper region of the vessel.

The preferred embodiments of the invention offer the advantage of particularly high conversions of feed gas in relatively simple equipment, in combination with the possibility of a high throughput. Further advantages which may be achieved by using this embodiment are that the temperature of the final section of the fluidized bed and, consequently, the temperature of the effluent of the reactor can be lower than the mean temperature of the total reactor. This results in a higher temperature and, consequently, a higher reaction rate in the first section(s) of the fluidized bed and a greater level of conversion in the subsequent section(s) of the bed.

An advantage offered by the process according to the present invention in general is that it is possible to replenish the catalyst while the process is in operation. Thus, catalyst stability requirements may, in part, be sacrificed in favour of a level higher level of catalyst activity. The synthesis reaction may thus be effected at higher temperatures in the first stage(s) of the process, thereby providing additional capacity to improve the space-time yield of the process.

The present invention is further illustrated by the attached figures, in which:

Figure 1 represents a schematic flowsheet of a process configuration for carrying out the process of the invention; and

Figure 2 represents an alternative process configuration of the process of the present invention.

The process configuration shown in each of the attached figures comprises three reactors ($R_1$, $R_2$ and $R_3$) arranged in series, each reactor comprising catalyst retained in a fluidized bed. The reactors are each equipped with at least one heat exchanger, an inlet for the feed gas and an outlet for the reactor effluent. The outlet of each reactor is connected to a heat exchanger (indicated as $E_1$, $E_2$, and $E_3$ for the reactors $R_1$, $R_2$ and $R_3$ respectively), which in turn is connected to a gas/liquid (methanol) separator (indicated as $S_1$, $S_2$, and $S_3$ for the reactors $R_1$, $R_2$ and $R_3$ respectively), having an outlet for unconverted feed gas and an outlet for the recovered liquid fraction. The reactors ($R_1$, $R_2$ and $R_3$), together with their associated heat exchangers and separators, are connected in series, the arrangement being such that the process operates as follows:

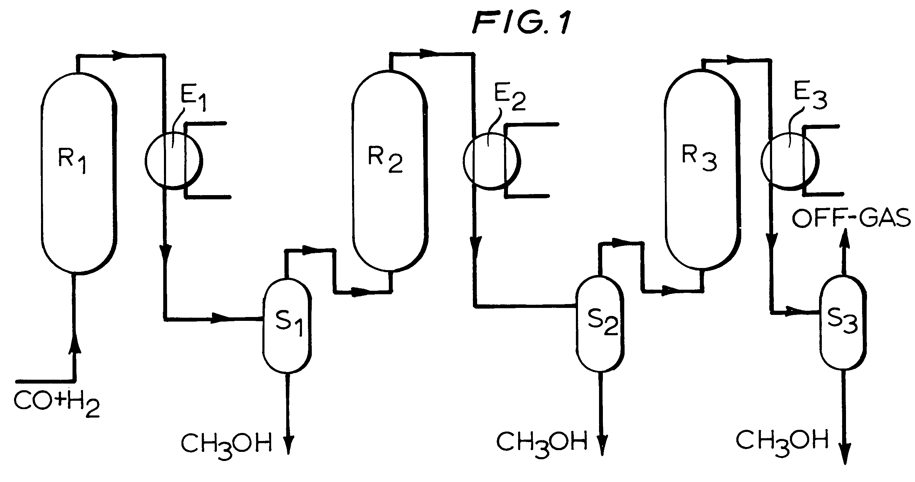

In the process of Figure 1, a fresh feed gas comprising hydrogen and carbon monoxide is fed directly to reactor $R_1$. The effluent of reactor $R_1$ is cooled in exchanger $E_1$ and passed directly to separator $S_1$, from which is recovered a first methanol product stream. The unconverted feed gas is passed to reactor $R_2$. The effluent of reactor $R_2$ is cooled in exchanger $E_2$ and a second methanol product stream recovered from separator $S_2$. The remaining unconverted feed gas is processed again in a similar manner in reactor $R_3$, exchanger $E_3$ and separator $S_3$, yielding a third methanol product stream and an off-gas.

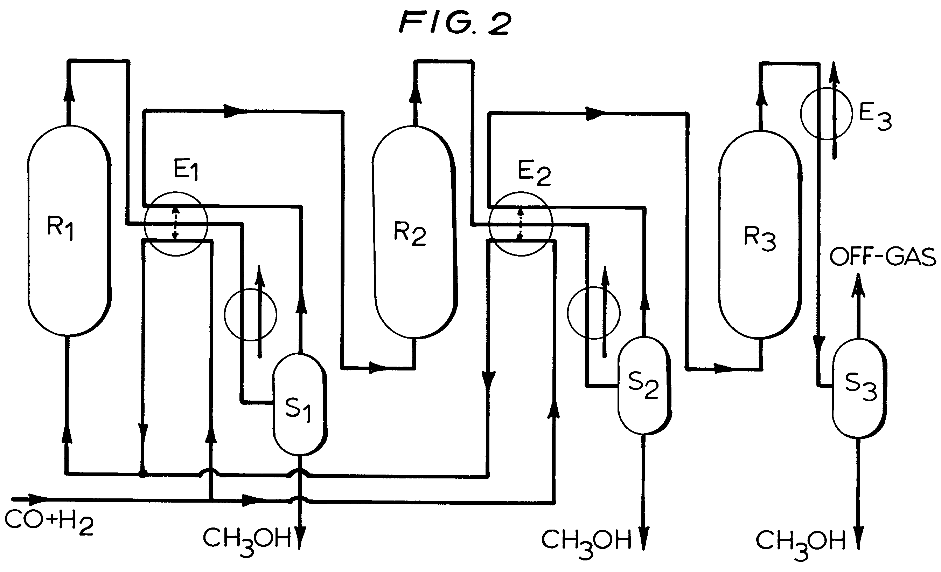

In the process of Figure 2, a fresh, cold feed gas, comprising a mixture of hydrogen and carbon monoxide, is split into two fractions, the fractions are preheated in heat exchangers $E_1$ and $E_2$ and recombined prior to being fed to the feed gas inlet of reactor $R_1$. The effluent from reactor $R_1$, comprising a mixture of methanol and unconverted feed gas, is passed via the outlet of reactor $R_1$ to heat exchanger $E_1$ and, thereafter to separator $S_1$. Liquid methanol is removed from separator $S_1$ as a product. The unconverted feed gas remaining is preheated in exchanger $E_1$ prior to being fed to the inlet of reactor $R_2$. The processing of the feed gas occurring in units $R_1$, $E_1$ and $S_1$ is repeated in units $R_2$, $E_2$ and $S_2$. A second methanol product stream is removed from the separator $S_2$ and the remaining unconverted feed gas heated in exchanger $E_2$ prior to being fed to the inlet of reactor $R_3$. Finally, the effluent of reactor $R_3$ is cooled in exchanger $E_3$ and passed to the separator $S_3$, from which a third methanol product stream is recovered. Unconverted feed gas leaves the process as off-gas. In exchanger $E_1$, heat is removed from the hot effluent of reactor $R_1$ and used to preheat both a fraction of the feed for reactor $R_1$ and the feed for reactor $R_2$. A similar duty is performed by exchanger $E_2$. Additional cooling of the effluent streams of reactors $R_1$ and/or $R_2$ may be required to achieve the required level of methanol recovery in separators $S_1$ and $S_2$. This may be achieved in heat exchangers shown in the figure (unlabelled).

By selecting suitable reaction conditions and an active catalyst, for example a catalyst comprising reduced $Cu-ZnO-Cr_2O_3$ and having a particle size below 0.1 mm, it is possible to obtain a degree of conversion of more than 60% per pass reactor stage in the process. After multiple stages, for example three stages as illustrated, an overall yield of at least 90% of the feed gas to the first reactor can be achieved. A methanol purity of over 98% can be obtained.

It will be appreciated that, in processes in which the feed gas to the first reactor comprises greater than 2 moles of hydrogen per mole of carbon monoxide, the off-gas produced after three or more stages is rich in hydrogen. By applying membrane separation techniques to this hydrogen rich gas it is possible to obtain chemically pure hydrogen.

The process of the present invention is further illustrated in the following typical process scenario:

A feed gas having a molar composition of 67% $H_2$, 32% CO and 1% $CO_2$ is converted into methanol in three reactors in series. Each reactor contains a fluidized bed of catalyst in which cooling tubes are arranged to remove the reaction heat. Boiler feed water passing through the tubes is partly converted into saturated high pressure steam and the mixture of water and steam may be separated in a steam drum. The steam produced is used as process steam and the water recycled to the inlet of the cooling tubes. The pressure of the steam generation circuit is set according to the desired operating temperature in the fluidized bed.

Each of the reactors is filled with a catalyst comprising copper, zinc and chromium in the molar ratio 25/48/27. The catalyst is in the form of microspheres of an average diameter of 50 microns. The catalyst may be prepared according to the procedure disclosed in European Patent Application publication No. 0 109 703. The quantity of catalyst retained in the first, second and third reactors is in the weight ratio 100:45:20 respectively. Before use, the catalyst in each reactor is activated by reduction in a flow of hydrogen gas at a pressure of from 100 to 500 kPa and 220 °C.

The gaseous mixture used as feed is preheated to 230 °C and fed to the bottom of the first reactor. The gas space velocity in the first reactor is about 3500 $Nm^3$ per $m^3$ of settled catalyst per hour. The inlet pressure of the first reactor is 8 MPa and the average bed temperature is 250 °C. The reactor effluent leaves the top of the reactor and has a molar composition of 45.1% $H_2$, 21.3% CO, 0.8% $CO_2$ and 32.8% $CH_3OH$. The effluent is cooled to about 30 °C and led to a gas-liquid separator where the major part of the methanol formed is separated off as liquid methanol. The unconverted feed gas stream leaving the separator has a molar composition of 66.4% $H_2$, 31.4% CO, 1.2% $CO_2$ and 1% $CH_3OH$. The unconverted feed gas stream is heated to about 230 °C and fed to the bottom of the second reactor. The second reactor is operated at an inlet pressure of 7.5 MPa, an average temperature of 250 °C and a gas space velocity of 3200 $Nm^3$ per $m^3$ of settled catalyst per hour.

The reactor effluent from the top of the second reactor has a molar composition of 44.6% $H_2$, 20.7% CO, 1.0% $CO_2$ and 33.7% $CH_3OH$. the effluent is cooled to about 30 °C and led to a second gas-liquid separator where the major part of the methanol is separated off as liquid methanol. The unconverted feed gas stream leaving the second separator has a molar composition of 66.5% $H_2$, 30.8% CO, 1.5% $CO_2$ and 1.2% $CH_3OH$. The unconverted feed gas stream is heated to about 230 °C and fed to the bottom of the third reactor. The third reactor is operated at an inlet pressure of 7 MPa, an average temperature of 250 °C and a gas space velocity of 2900 $Nm^3$ per $m^3$ settled catalyst per hour. The reactor effluent leaving the top of the third reactor has a molar composition of 49.9% $H_2$, 22.2% CO, 1.3% $CO_2$ and 26.5% $CH_3OH$. The effluent is cooled to about 30 °C and led to a third gas-liquid separator where the major part of methanol is withdrawn as liquid methanol.

The operation of the process according to the above scenario could yield the following typical results:

The conversion of CO in the first, second and third reactors is 60, 60 and 58%, respectively. The total conversion of CO is 93%. The quantities of methanol withdrawn from the first, second and third separator are 19.3, 8.1 and 2.4 moles per 100 moles of original feed gas. The total amount of methanol yielded is 29.8 moles per 100 moles of feed gas, which represents 90.3% of the theoretical yield.

**Claims**

1. A process for the preparation of methanol, characterised in that a gaseous mixture comprising hydrogen and carbon monoxide is reacted in the presence of a catalyst in a plurality of sequentially arranged stages, the gaseous mixture contacting the catalyst in each stage in a fluidized bed regime whilst being cooled, in which process methanol is removed from the reaction mixture between successive stages.

2. A process according to claim 1, characterized in that the interstage removal of methanol is carried out by cooling the effluent stream from the preceding reactor and allowing condensation of the methanol to occur.

3. A process according to claim 2, characterized in that the cooling is effected by heat exchange of the effluent from the reactor with feed gas.

4. A process according to claim 3, characterized in that the heat exchange between the effluent from the reactor and the feed gas is effected using a heat exchanger having a specific heat transfer area of at least 150 $m^2/m^3$, preferably of at least 200 $m^2/m^3$.

5. A process according to any preceding claim, characterized in that the fluidized bed is divided in a plurality of interconnected fluidized bed sections arranged in series, in which each section is cooled by at least one heat exchanger.

6. A process according to claim 5, characterized in that the temperature of the final fluidized bed section encountered by the feed gas is maintained at a lower temperature than the highest temperature of the preceding sections.

7. A process according to claim 6, characterized in that the final section is maintained at an average reaction temperature which is from 10 to 100 °C lower than the highest temperature in the preceding sections.

8. A process according to any preceding claim, characterized in that the catalyst composition in the fluidized bed comprises copper and zinc, optionally promoted with another element.

9. A process according to any preceding claim, characterized in that a portion of the catalyst is replenished during the operation of the process.

10. A process according to any preceding claim, characterized in that the feed gas to the process comprises carbon monoxide and hydrogen in a molar ratio $H_2$:CO in the range of from 1 to 3, preferably from 1.5 to 2.5.

11. Methanol whenever produced by a process according to any preceding claim.

FIG.1

FIG. 2